# EUROPEAN PATENT APPLICATION

(11) **EP 4 265 330 A1**
(43) Date of publication of application: **25.10.2023**
(21) Application number: 22169437.5
(22) Date of filing: 22.04.2022
(51) Int. Cl.: B01L 3/00, G01N 33/14, B65D 81/38, A47G 19/22

(54) **TESTING CONTAINER**

(71) Applicant: BIC Violex Single Member S.A., 145 69 Anoixi (GR)
(72) Inventor: Chrysanthakopoulos, Nikolaos, 14569 Anoixi (GR)
(74) Representative: Peterreins Schley

(57) **Abstract**

There is provided a smart beverage container for analyzing saliva. The smart beverage container comprises a body comprising a top edge with a saliva absorbing rim area configured to collect saliva of a user, a biosensor coupled to the body and configured to receive power from a power source, and a microfluidic network between the saliva absorbing rim area and the biosensor, wherein the microfluidic network comprises a plurality of channels configured to direct the collected saliva to the biosensor, wherein the biosensor is configured to analyze saliva and to provide the result of the analysis to a communication interface.

## Description

### TECHNICAL FIELD

The embodiments described herein relate to a smart beverage container for analyzing saliva, an associated computer-implemented method for visualizing a saliva analysis result, and a method for manufacturing the smart beverage cup.

### BACKGROUND

In the consumer goods industry in particular, research is increasingly focusing on the area of health monitoring for private individuals, which anyone can carry out themselves regardless of medical indications. The emergence of so-called wearables has been a particular driver of this development. Various sensors integrated into smartwatches, for example, measure heartbeat, body temperature, or record movement patterns. That people have an increasing focus on their health is here more reason than the result of the development. One result of the research is the emergence of biosensors, which are increasingly inexpensive to manufacture and can be operated with low power consumption. Biosensors are increasingly enabling the accurate analysis of body fluids, such as sweat, tears, saliva, or blood. Biosensors can measure the amount of critical metabolites, such as lactate or glucose, opening up possibilities for health monitoring down to the cellular level.

The development of health monitoring was then particularly accelerated by the emergence of the Corona pandemic. During the pandemic, the need for inexpensive, rapid, and convenient ways to test oneself for SARS-CoV-2 has arisen. A need has also emerged for area-wide testing of many people that is rapid and inexpensive. PCR tests and rapid antigen tests by throat swab are common ways to detect SARS-CoV-2 infection. So-called "saliva or spit tests," which test for the virus in saliva, have also become common. Also, in general, and independent of the Corona pandemic, there is a growing desire to take care of one's health, and also to prevent infecting other people with possible diseases. Thus, there is a need for fast, inexpensive, and convenient testing options that on the one hand can be easily integrated into everyday life, but also reliably test for various viruses, bacteria, or other indicators that suggest disease and/or infection.

Finding a way to integrate testing options into everyday life in a convenient manner can lead to a variety of approaches.

Even before the Corona pandemic, but especially during it, drinking hot beverages such as coffee, tea, or hot chocolate from to-go cups, for example from the coffee shop or taken from home, became established in the population. Disposable coffee cups also continue to be widely used. Recent surveys estimate the market size for single-use out-of-home (OOH) hot paper coffee cups at 118 billion units per year with a compounded annual growth rate of 1.8% to reach 294 billion units by 2025.

The preceding facts about beverage containers combined with the need for testing options in everyday life, lead to the situation that beverage containers can be further developed to use the beverage containers for health monitoring.

### SUMMARY

According to a first aspect, there is provided a smart beverage container for analyzing saliva. The smart beverage container comprises a body comprising a top edge with a saliva absorbing rim area configured to collect saliva of a user, a biosensor coupled to the body and configured to receive power from a power source, and a microfluidic network between the saliva absorbing rim area and the biosensor, wherein the microfluidic network comprises a plurality of channels configured to direct the collected saliva to the biosensor, wherein the biosensor is configured to analyze saliva and to provide the result of the analysis to a communication interface.

According to a second aspect, there is provided a computer-implemented method for visualizing a saliva analysis result. The computer-implemented method comprises receiving, via a user interface, a selection of one or more test protocols selected by the user from a plurality of test protocols, enabling the communication with a communication interface of a smart beverage container, receiving, from the communication interface, results of a saliva analysis processed by a sensor of the beverage container, and visualizing the results on a display device. According to a third aspect, there is provided a method for manufacturing a smart beverage container. The method comprises providing a body comprising a top edge with a saliva absorbing rim area configured to collect saliva, coupling a biosensor to the body configured to analyze saliva, coupling a microfluidic network between the saliva absorbing rim area and the biosensor configured to direct the collected saliva to the biosensor, coupling a power source to the body configured to provide electrical power, and coupling a communication interface to the body.

An effect of the technique of the present specification is to provide a testing apparatus that can be integrated into everyday life in such a way that time-consuming and inconvenient, and also costly, testing alternatives may no longer be essential. It can also increase the frequency of testing, as the use of a smart beverage container takes place in everyday life anyway, and thus the convenience of to-go beverages can be combined with simultaneous testing for vital sign metrics or chronic or acute illness biomarkers. Furthermore, testing saliva, which is produced anyway when drinking, may be a convenient alternative to possibly painful procedures which can be necessary to test a person for various infections or diseases.

As beverage containers are also widely used at large events or festivals, smart beverage containers can help to test large crowds for possible infections. The smart beverage container provides an everyday object for saliva testing and can thus enable low-threshold testing for infections or diseases that would otherwise not be performed or only with appropriate indication by a treating physician or other instructions.

The configuration of the smart beverage container described herein allows a low-cost production. The use of inexpensive components makes it possible to configure a reusable beverage container as well as a disposable beverage container, such as might be found in coffee shops or at large events.

Furthermore, regular testing can contribute to a kind of health monitoring, so that slight changes in the user's state of health can be identified.

### BRIEF DESCRIPTION OF THE DRAWINGS

Other characteristics will be apparent from the accompanying drawings, which form a part of this disclosure. The drawings are intended to further explain the present disclosure and to enable a person skilled in the art to practice it. However, the drawings are intended as non-limiting examples. Common reference numerals on different figures indicate like or similar features.
- **Figure 1**: schematically illustrates an example of a smart beverage container with an optional display device.
- **Figure 2**: schematically illustrates an example of a smart beverage container with a wireless power transfer coil as a power source.
- **Figure 3**: schematically illustrates an example of a smart beverage container with a thermoelectric generator film as a power source.
- **Figure 4**: schematically illustrates an example of a smart beverage container with a body comprising two walls.
- **Figure 5**: schematically illustrates an example of a computer-implemented method for visualizing a saliva analysis result according to the second aspect.

### DETAILED DESCRIPTION

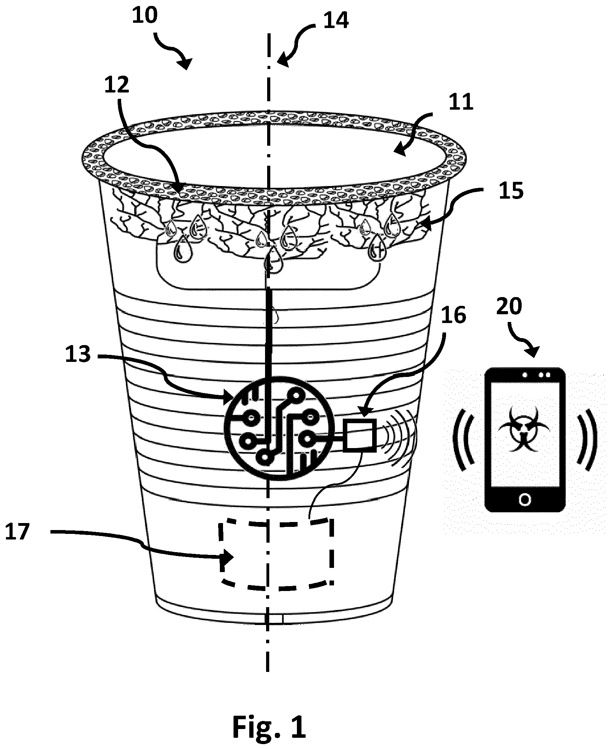

**Fig. 1** schematically illustrates an example of a smart beverage container 10. According to the first aspect, the smart beverage container 10 for analyzing saliva comprises a body 11 comprising a top edge with a saliva absorbing rim area 12 configured to collect saliva of a user, a biosensor 13 coupled to the body 11 and configured to receive power from a power source 18, 19, and a microfluidic network 15 between the saliva absorbing rim area 12 and the biosensor 13. Further, the microfluidic network 15 comprises a plurality of channels configured to direct the collected saliva to the biosensor 13, wherein the biosensor 13 is configured to analyze saliva and to provide the result of the analysis to a communication interface 16.

In an example, the communication interface 16 may be configured to communicate with a mobile device 20 or a stationary computer unit to transmit the analysis results from the biosensor 13. The communication interface 16 may be electrically connected to the biosensor 13 to receive a signal corresponding to the saliva analysis result from the biosensor 13. The communication interface 16 may be configured to store the signal corresponding to the saliva analysis result from the biosensor 13. The mobile device 20 (or the stationary computer unit) may be configured to receive the result of the analysis and may be configured to visualize the result of the analysis using the computer-implemented method 100 according to the second aspect. The communication interface 16 may be a passive chip coupled to the body 11 of the beverage container 10. The communication interface 16 may enable wireless communication like near-field communication, Wi-Fi, and/or Bluetooth. The communication interface 16 may be a near-field communication chip (NFC). The near-field communication chip may be of type 1, 2, 3, 4, 5 according to the NFC standard, or Mifare Classic. The communication interface 16 may enable a connection with a mobile device 20 within a specified range, for example up to 10 cm, up to 10 m, or up to 100 m. The communication interface 16, like a near-field communication chip, may be configured to communicate with the mobile device 20 using a signal with a data frequency of 13.56MHz. Further, the communication interface 16 may be configured to communicate with a mobile device 20 using a data transfer speed ranging from 106 to 424 kbit/s. The communication interface 16 may be configured to operate in peer-to-peer mode, a card emulation mode, or a read/write mode. The communication interface 16 may be configured for wireless power transfer and to receive electrical power from the mobile device 20 or stationary computer. The communication interface 16 may be configured to provide electrical power to the biosensor 13. The communication interface 16 may be configured to receive data, for example, display instructions for a display device 17, from the mobile device 20 or stationary computer. As an example, the communication interface 16 may transmit the analysis results from the biosensor 13 to the mobile device 20 or stationary computer, and then receives data for further process steps, for example, display instructions for the display device 17, corresponding to the transmitted analysis results from the mobile device 20 or stationary computer.

**Fig. 1** schematically illustrates an example of a smart beverage container 10 further comprising a display device 17.

In an embodiment, the smart beverage container may comprise a display device 17 on an external side of the body 11 being configured to visualize the result of the analysis. In an example, when an analysis of the saliva comprises a test leading to a positive or a negative result, the display device 17 may be configured to show if a test is negative or positive. For example, the display device 17 may be configured to display a relevant symbol and/or a text message corresponding to the saliva analysis result from the biosensor 13. In an example, the display device 17 may be one of a liquid crystal display, an organic light emitting diode display, an active-matrix organic light-emitting diode display, or a light emitting diode display. In another example, the display device 17 may comprise an electronic paper display. For example, display device 17 may be one of a microencapsulated electrophoretic display device, an electrowetting display device, an electrofluidic display device, or a plasmonic electronic display device. The display device 17 may be configured to receive display instructions, such as a driving signal and/or driving data, from the communication interface 16. In an example, the display device 17 may be configured to get refreshed by the mobile device 20 through the communication interface 16, wherein refreshing comprises stopping visualizing a preceding symbol and/or text message and starting visualizing a subsequent symbol and/or text message. In an example, a preceding symbol and/or text message may correspond to a first saliva analysis result and a subsequent symbol and/or text message may correspond to a second saliva analysis result. In an example, the display device 17 may be configured to receive electrical power from the communication interface 16, which may be configured for wireless power transfer and to receive electrical power from the mobile device 20 or a stationary computer and to provide electrical power to the display device 17.

In an embodiment, the biosensor 13 may be configured to detect one or more biomarkers, viruses, and/or bacteria. In an example, the biosensor 13 may be configured to analyze saliva through enzyme-based electrocatalysis. The biosensor 13 may be configured to provide, as a result, a signal corresponding to the detection of one or more biomarkers, viruses, and/or bacteria to the communication interface 16. The biosensor 13 may be connected to the microfluidic network 15 and may be configured to receive saliva of the user collected by the saliva absorbing rim area 12 and directed via the channels of the microfluidic network 15 to the biosensor 13.

In an embodiment, the biosensor 13 may be one of an amperometric biosensor, an organic electrochemical transistor (OECT), or an all-polymer micrometer-scale transistor biosensor. One example of an organic electrochemical transistor (OECT) may be p-type semiconductor poly(3,4-ethylenedioxythiophene) doped with polystyrene sulfonate (PEDOT:PSS) being coupled with the corresponding oxidase enzymes for metabolite sensing. One example for a polymer biosensor may be a n-type-conjugated polymer as the active material based on an NDI-T2 copolymer, which may have a backbone comprising a highly electron-deficient naphthalene-1,4,5,8-tetracarboxylic diimide (NDI) repeat unit and an electron-rich unsubstituted bithiophene repeat unit (T2) called P-90. The side chains on the diimide unit may be a 90:10 randomly distributed ratio of polar glycol and nonpolar branched alkyl groups, where the ratio may be optimized to ensure solubility of the copolymer in polar solvents. The polymer biosensor, as an electrolyte-gated transistor, may be able to transduce ionic signals of biological origin into electronic ones, with high amplification. The polymer biosensor may comprise an n-type organic polymer semiconductor, an ion-to-electron converting device as the organic electrochemical transistor. The n-type polymer may incorporate hydrophilic side chains to enhance ion transport/injection, as well as to facilitate enzyme conjugation. The material may be capable of accepting electrons of the enzymatic reaction and act as a series of redox centers capable of switching between the neutral and reduced state resulting in a fast, selective, and sensitive metabolite sensor. In an example, the biosensor 13 may measure the amount of critical metabolites, such as lactate or glucose.

In an embodiment, the one or more biomarkers may comprise endocrine biomarkers like cortisol, testosterone, and/or insulin, immunologic biomarkers like IgA, IgM, and/or IgG, inflammatory biomarkers like cytokines, proteins like enzymes or antibodies, infectious or pathogen RNA, metabolites like vitamins, and/or cancer biomarkers. In an example, a voltage needed for the operation of the biosensor 13 may range from 200mV to 1000mV. In an example, the current needed for the operation of the biosensor 13 may range from 0.5µA to 3µA. In an example, the power needed for the operation of the biosensor 13 may range from 0.1mW to 3mW. In an example, a transistor size of the biosensor 13 may range from 10µm to 200µm. In an example, a process size or a gate length of a transistor of the biosensor 13 may range from 10µm to 200µm. In an example, the thickness of the biosensor 13 may range from 100µm to 1000µm.

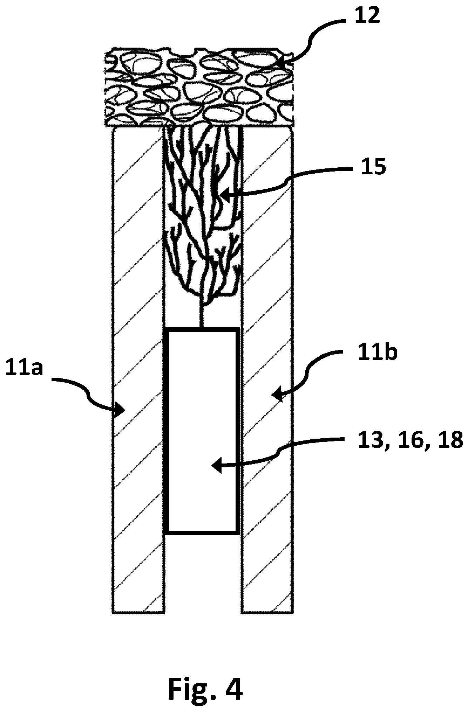

**Fig. 4** schematically illustrates an example of a saliva absorbing rim area 12 comprising a plurality of pores in greater detail.

In an embodiment, the saliva absorbing rim area 12 may comprise a plurality of pores microfluidically coupled to an upper end of the channels of the microfluidic network 15.

The saliva absorbing rim area 12 may be configured to collect saliva of the user through pores and to direct the saliva to the biosensor 13 using gravity via the microfluidic network 15. In an example, the saliva absorbing rim area 12 may be made of a material comprising at least one of high-density polyethylene (HDPE), low-density polyethylene (LDPE), polycarbonate (PC), polyethylene terephthalate (PET), polypropylene (PP), paper with a polyethylene coating, ceramic and/or sponge. In an example, the saliva absorbing rim area 12 may comprise a removable plastic film seal or paper seal covering the saliva absorbing rim area 12. For example, during transport of the beverage container 10 or during dispensing, for example in a coffee shop, the rim area 12 may become contaminated, and non-desirable debris and/or pathogens from third parties can be passed to the biosensor 13 with the saliva of the user leading to an analysis result not reflecting the actual health profile of the user. For another example, a seal may prevent pollution and/or clogging of the pores of the saliva absorbing rim area 12. In an example, the saliva absorbing rim area 12 may be made of a material comprising at least one of a natural and/or artificial microfluidic porous material. In an example, the pores of the plurality of pores may be orderly distributed or may be randomly distributed in the saliva absorbing rim area 12. In an example, the pores of the plurality of pores may have a size ranging from 30µm to 300µm. In an example, the pores may have circular, rectangular, or elliptical cross-sections or may be slit-shaped openings. In an example, the pores may have a circular cross-section with a diameter ranging from 30µm to 300µm. In another example, the pores may have an elliptical cross-section with a major axis ranging from 30µm to 300µm or a minor axis ranging from 30µm to 300µm. In an example, the pores may have a rectangular cross-section with an edge length ranging from 30µm to 300µm or a diagonal ranging from 30µm to 300µm. In another example, the thickness of the saliva absorbing rim area 12 may be in the range of 1mm to 10mm wherein a thickness direction may be in a direction perpendicular to a longitudinal axis 14 of the body 11. In another example, the height of the saliva absorbing rim area 12 may be in the range of 3mm to 10mm wherein a height direction may be in a direction parallel to the longitudinal axis 14 of the body. In an example, the saliva absorbing rim area 12 may extend around a part of the perimeter of the top edge of the body 11. In another example, the saliva absorbing rim area 12 may extend around the entire perimeter of the top edge of the body 11.

**Fig. 4** schematically illustrates an example of microfluidic network 15 in greater detail.

In an embodiment, the microfluidic network 15 may have a dendritic distribution, the upper end comprising a higher number of channels being connected to the saliva absorbing rim area 12 and a lower end comprising a lower number of channels being connected to the biosensor 13. The cross-section of the channels may be circular, rectangular, or elliptic. The channels with circular cross-sections may have diameters being equal or diameters being different. The channels with rectangular cross-sections may have edge lengths being equal or edge lengths being different. The channels with elliptic cross-sections may have major axis lengths being equal or major axis lengths being different. The channels with circular cross-sections may have diameters ranging from 10µm to 3mm. The channels with rectangular cross-sections may have edge lengths ranging from 10µm to 3mm. The channels with elliptic cross-sections may have major axis lengths ranging from 10µm to 3mm. In an example, the diameters of the channels being closer to the upper end may be smaller than the diameters of the channels being closer to the lower end. Saliva collected by the saliva absorbing rim area 12 is directed into the channels of the microfluidic network 15 connected to the saliva absorbing rim area 12. The microfluidic network 15 may comprise a plurality of branches, wherein at each branch, two or more channels join to form a subsequent channel. During using the smart beverage container 10, the amount of saliva may become larger in the subsequent channel after the branch. The saliva received by a higher number of channels from the saliva absorbing rim area 12 may be directed into the biosensor 13 by a lower number of channels connected to the biosensor 13. The microfluidic network 15 may be bifurcating and/or orderly distributed around the perimeter of the body 11. In an example, a higher number of channels of the microfluidic network 15 run parallel to each other beginning form the saliva absorbing rim area 12 to join to a lower number of channels in front of the biosensor 13. In an example, the microfluidic network 15 may be made of a material comprising at least one of glass, silicon, polymer (PDS), and/or paper. In an example, the microfluidic network 15 may be distributed all along the perimeter of the upper part of the body 11. In another example, the microfluidic network 15 may be distributed along a part of the perimeter of the upper part of the body 11. The microfluidic network 15 may cover a part of the perimeter of the body 11 corresponding to the part of the perimeter of the top edge of the body 11 covered by the saliva absorbing rim area 15. In an example, the microfluidic network 15 may be coupled to the body 11 on the external or internal side, such as by an adhesive connection. **Fig. 4** illustrates schematically an example in which the body comprises an external wall 11a and an internal wall 11b, wherein the microfluidic network 15 is coupled to the body 11 between the external wall 11a and the internal 11b.

In an embodiment, the power source may comprise a thermoelectric generator film 18 and/or a wireless power transfer coil 19 configured to receive power from the mobile device 20.

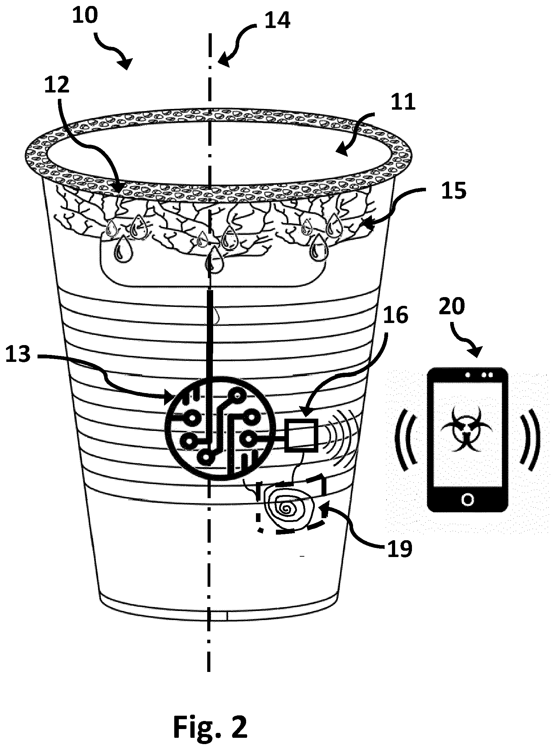

**Fig. 2** schematically illustrates an example of a smart beverage container comprising a wireless power transfer coil 19.

The wireless power transfer coil (WPT) 19 may be configured to receive power from a mobile device 20 or a stationary computer through inductive coupling, capacitive coupling, magnetodynamic coupling, and/or Zenneck wave transmission. The wireless power transfer 19 may be configured to provide electrical power to the biosensor 13, the communication interface 16, and/or to the display device 17. The wireless power transfer 19 may be an additional component and/or may be implemented as part of the communication interface 16. In an example, a near-field communication chip may comprise a wireless power transfer coil 19.

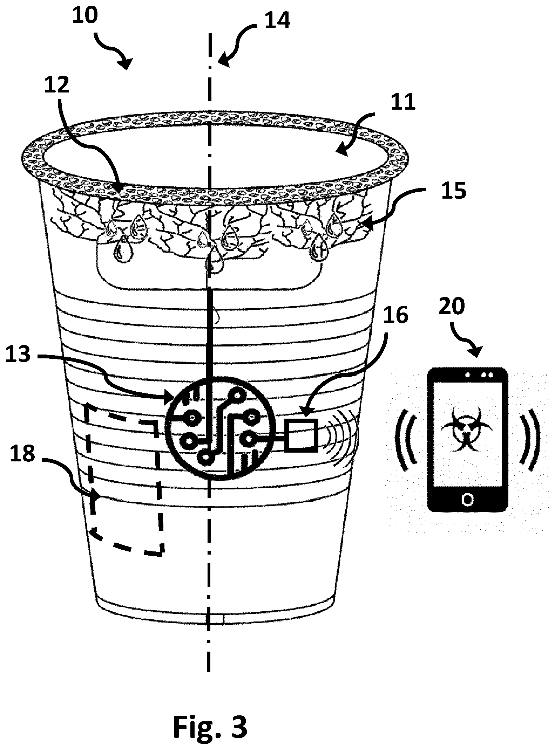

**Fig. 3** schematically illustrates an example of a smart beverage container comprising a thermoelectric generator film 18.

In an embodiment, the thermoelectric generator film 18 may be configured to generate electrical power using the heat of beverage inside the container 10 having a temperature higher than the environmental temperature outside the container 10. When using the smart beverage container 10, the user may pour hot beverage into the container 10. The thermoelectric generator film 18 may use the Seebeck effect or the Nernst effect to generate power using the temperature difference between the hot beverage inside the container 10 and the environmental temperature outside the container when the container is used by the user. The thermoelectric generator film 18 may be made of a material comprising at least one of a semiconductor type like bismuth telluride, lead telluride, and/or silicon germanium, inorganic clathrates, compounds of Mg and group-14 element, skutterudites, cation-substituted copper sulfide thermoelectrics, Half-Heusler alloys, silicon-germanium, sodium-cobaltate, amorphous materials, functionally graded materials, tin selenide, silicon-nanowire, and/or iron-doped with aluminium (Fe₃Al) or gallium (Fe₃Ga). An example of a nontoxic thermoelectric generator film may be made by alpha iron doped with aluminum (Fe₃Al) or gallium (Fe₃Ga) and may be based on the Nernst effect. The foregoing material may have an efficiency of 6µV/K and may be flexible and/or more effective than a conventional thermoelectric generator film material based on the limited Seebeck effect. The efficiency of the iron-doped thermoelectric generator film may range from 3µV/K to 9µV/K. The power delivered by the iron-doped thermoelectric generator film may range from 0.5mW to 2mW.

In an embodiment, the thermoelectric generator film 18 may be located on the internal side of the body 11 or may be located on the external side of the body 11. The thermoelectric generator film 18 may cover part or all of the surface of the internal side of the body 11 or may cover part or all of the surface of the external side of the body 11. In an example, the thickness of the thermoelectric generator film 18 may range from 0.1mm to 2mm. The thermoelectric generator film 18 may electrically be connected to the biosensor 13, the communication interface 16, and/or the display device 17 for providing electrical power.

**Fig. 4** schematically illustrates an example of a smart beverage container with a body 11 comprising two walls 11a, 11b.

In an embodiment, the body 11 may comprise at least two walls 11a, 11b. The biosensor 13, communication interface 16, the microfluidic network 15, and/or the thermoelectric generator film 18 may be located between two of the at least two walls 11a, 11b. In an example, the body 11 may comprise an air gap between two of the at least two walls 1 1a, 11b. In another example, the body 11 may be made of a material comprising at least one of a biodegradable, compostable, degradable, and/or recyclable material. In another example, the body 11 may be made of a material comprising at least one of plastic (PS, PP, PET, EPS, PC), bioplastic, paper, wood, glass, ceramic, aluminum, and/or cork and/or may be polycoated. In an example, the body 11 may comprise an insulating layer on one or both sides of the body 11. If a body 11 comprises at least two walls 11a, 11b, the insulating layer may be between two of the at least two walls as an insulating middle layer. A corrugated medium may be used as a middle layer to add structural rigidity and thermal insulation. Additional thermal insulation may be induced, either by including an air gap or by incorporating polymeric insulating stripes between the outer wall 11a and inner wall 1 1b of the body. The insulating layer may extend over the entire circumferential surface of the body or only a part of it. In an example, the shape of the beverage container and/or the body may be conical or tubular. In an example, the diameter of a circular cross-section at the top edge of the body 11 equals the diameter of the cross-section of the base of the body 11. In another example, the diameter of a circular cross-section at the top edge of the body 11 is smaller than the diameter of the cross-section of the base of the body 11. In another example, the diameter of a circular cross-section at the top edge of the body 11 is larger than the diameter of the cross-section of the base of the body 11. The diameter of a base of the body 11 may be in the range of 40mm to 90mm. The height of the body 11 may range from 50mm to 180mm. The beverage holding capacity volume may range from 200ml to 600ml. In the case of a body 11 comprising at least paper, the paper may have a weight of 10-20 g/m². The weight of the body 11 may range from 5gr to 50gr. The beverage container may contain a holder and/or a sleeve encircling part of the height of the body 11. In an example, a lid or a cap may be attached to the top edge of the body 11. The body 11 may be a cup, a bottle, or a thermos.

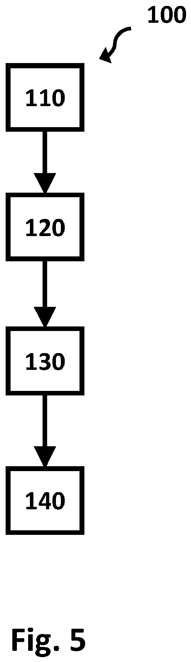

**Fig. 5** schematically illustrates an example of a computer-implemented method for visualizing a saliva analysis result according to the second aspect.

According to the second aspect, there is provided a computer-implemented method 100 for visualizing a saliva analysis result. The method comprises receiving 110, via a user interface, a selection of one or more test protocols selected by the user from a plurality of test protocols. The method further comprises enabling 120 the communication with a communication interface 16 of a smart beverage container 10. The method further comprises receiving 130, from the communication interface 16, results of a saliva analysis processed by a biosensor 13 of the beverage container 10. The method further comprises visualizing 140 the results on a display device. The user interface may be part of an application being executed on the mobile device 20 or a stationary computer. The user interface may be used to identify a specific smart beverage container 10 and enable the powering of the biosensor 13 and the wireless communication of the mobile device 20 with the communication interface 16. The user interface may be configured to provide a selection of one or more test protocols to the user. The test protocols may comprise a selection of parameters and/or biomarkers. The biomarkers may comprise at least one of endocrine biomarkers like cortisol, testosterone, and/or insulin, immunologic biomarkers like IgA, IgM, and/or IgG, inflammatory biomarkers like cytokines, proteins like enzymes or antibodies, infectious or pathogen RNA, metabolites like vitamins, and/or cancer biomarkers. The display device may be part of the mobile device 20 or a stationary computer. Visualizing 140 the result on a display device may comprise visualizing one or more symbols and/or text messages corresponding to the saliva analysis result. The method further may comprise after receiving 130, from the communication interface 16, results of a saliva analysis, processing the analysis, and sending display instructions to the communication interface 16. Display instructions may comprise one or more symbols and/or text messages.

According to the third aspect, there is provided a method for manufacturing a smart beverage container 10. The method comprises providing a body 11 comprising a top edge with a saliva absorbing rim area 12 configured to collect saliva. **Fig. 4** schematically illustrates an example of a saliva absorbing rim area 12 in greater detail. The method further comprises coupling a biosensor 13 to the body 11 configured to analyze saliva. The method further comprises coupling a microfluidic network 15 between the saliva absorbing rim area 12 and the biosensor 13 configured to direct the collected saliva to the biosensor 13. The method further comprises coupling a power source 18, 19 to the body 11 configured to provide electrical power, and coupling a communication interface 16 to the body 11.

Further, there is provided a method for using a smart beverage container 10 for analyzing saliva. The method further may comprise providing a smart beverage container 10. The method further may comprise pouring hot beverage into the container 10. The method further may comprise removing a protective sealing film from a saliva absorbing rim area 15. The method further may comprise drinking a sip of the beverage. The saliva of the user, particularly from the interior of the lower lip of the user, may be absorbed by the saliva absorbing rim area 12. The saliva may be directed through the microfluidic network 15 connected to the saliva absorbing rim area 12 to the biosensor 13. The biosensor 13 analyzes the saliva of the user. The biosensor 13 may provide a signal to the communication interface 16, wherein the signal may correspond to a result of the analysis. The signal may be stored in the communication interface 16. The method further may comprise bringing the mobile device 20 in contact with a communication interface 16 coupled to the body 11 of the container. Bringing in contact may comprise bringing the mobile device 20 within a distance from the communication interface 16 such that a communication channel for transmitting data between the communication interface 16 and the mobile device 20 may be established by the mobile device 20 and/or the communication interface 16. Alternatively, the method may comprise bringing the communication interface 16 in contact with a mobile device 20 or a stationary computer. The mobile device 20 or a stationary computer may receive the saliva analysis result stored in the communication interface 16 and process the result. The mobile device 20 or a stationary computer may visualize the result on a display device and/or send display instructions corresponding to the processed analysis result to the communication interface 16. The display device 17 may receive the display instructions from the communication interface 16 and visualizes the display instructions corresponding to the analysis result. The method further may comprise reading the result directly from the display device 17 attached to the body 11 of the container 10 and/or reading the result from a display of the mobile device 20.

In an embodiment, there is provided a computer program element comprising machine readable instructions which, when executed by a processor, causes the processor to perform the computer implemented method according to the second aspect, or its embodiments. A processor may be part of the mobile device 20 or a stationary computer.

In an embodiment, there is provided a computer readable medium comprising the computer program element. The computer readable medium may be part of the mobile device 20 or a stationary computer.

References throughout the preceding specification to "one embodiment", "an embodiment", "one example" or "an example", "one aspect" or "an aspect" means that a particular feature, structure, or characteristic described in connection with the embodiment or example is included in at least one embodiment of the present disclosure. Thus, appearances of the phrases "in one embodiment", "in an embodiment", "one example" or "an example", "one aspect" or "an aspect" in various places throughout this specification are not necessarily all referring to the same embodiment or example.

Furthermore, the particular features, structures, or characteristics can be combined in any suitable combinations and/or sub-combinations in one or more embodiments or examples.

Embodiments
1. A smart beverage container 10 for analyzing saliva, comprising a body 11 comprising a top edge with a saliva absorbing rim area 12 configured to collect saliva of a user;
   a biosensor 13 coupled to the body 11 and configured to receive power from a power source 18, 19; and
   a microfluidic network 15 between the saliva absorbing rim area and the biosensor 13, wherein the microfluidic network 15 comprises a plurality of channels configured to direct the collected saliva to the biosensor 13;
   wherein the biosensor 13 is configured to analyze saliva and to provide the result of the analysis to a communication interface 16.
2. The smart beverage container according to embodiment 1, wherein the communication interface is configured to communicate with a mobile device.
3. The smart beverage container 10 according to embodiment 1 or 2, wherein the communication interface 16 comprises a near-field communication chip.
4. The smart beverage container 10 according to one of the preceding embodiments, wherein the communication interface is configured to communicate with a mobile device being within a range of 1cm to 10cm.
5. The smart beverage container 10 according to one of the preceding embodiments, wherein the communication interface is configured to communicate with a mobile device using a signal with a frequency of 13,56 MHz.
6. The smart beverage container 10 according to one of the preceding embodiments, wherein the communication interface is configured to communicate with a mobile device, wherein a data transfer speed ranges from 106 to 424 kbit/s.
7. The smart beverage container 10 according to one of the preceding embodiments, wherein the communication interface is configured to communicate with a mobile device with a peer-to-peer mode, a card emulation mode, and/or a reader/writer mode.
8. The smart beverage container 10 according to one of the preceding embodiments, further comprising a display device 17 located on an external side of the body 11 and configured to visualize the result of the analysis.
9. The smart beverage container 10 according to embodiment 8, wherein the display device 17 comprises an electronic paper display.
10. The smart beverage container 10 according to embodiment 8 or 9, wherein the display device 17 is one of a microencapsulated electrophoretic display device, an electrowetting display device, an electrofluidic display device, or a plasmonic electronic display device.
11. The smart beverage container 10 to embodiment 8, 9, or 10, wherein the display device (17) is configured to display a text message and/or a symbol corresponding to a saliva analysis result.
12. The smart beverage container 10 according to any one of the preceding embodiments, wherein the biosensor 13 is configured to detect one or more biomarkers, viruses and/or bacteria.
13. The smart beverage container 10 according to any one of the preceding embodiments, wherein the biosensor 13 is one of an amperometric biosensor, an organic electrochemical transistor (OECT), or an all-polymer micrometer-scale transistor biosensor.
14. The smart beverage container 10 according to embodiment 12, wherein the one or more biomarkers comprise endocrine biomarkers like cortisol, testosterone, and/or insulin, immunologic biomarkers like IgA, IgM, and/or IgG, inflammatory biomarkers like cytokines, proteins like enzymes or antibodies, infectious or pathogen RNA, metabolites like vitamins, and/or cancer biomarkers.
15. The smart beverage container 10 according to any one of the preceding embodiments, wherein the size of the biosensor is in the range of 10µm to 200µm.
16. The smart beverage container 10 according to any one of the preceding embodiments, wherein the thickness of the biosensor is in the range of 100µm to 1000µm .
17. The smart beverage container 10 according to any one of the preceding embodiments, wherein the saliva absorbing rim area 12 comprises a plurality of pores microfluidically coupled to an upper end of the channels of the microfluidic network.
18. The smart beverage container 10 according to any one of the preceding embodiments, wherein the saliva absorbing rim area 12 is made of a material comprising high-density polyethylene (HDPE), low-density polyethylene (LDPE), polycarbonate (PC), polyethylene terephthalate (PET), polypropylene (PP), paper with a polyethylene coating, ceramic, sponge.
19. The smart beverage container 10 according to any one of the preceding embodiments, wherein the saliva absorbing rim area 12 comprises a removable plastic film seal or a paper seal covering the saliva absorbing rim area.
20. The smart beverage container 10 according to any one of the preceding embodiments, wherein the saliva absorbing rim area 12 is made of a material comprising a natural and/or artificial microfluidic porous material.
21. The smart beverage container 10 according to any one of embodiments 17 to 20, wherein the pores of the plurality of pores are orderly distributed or are randomly distributed in the saliva absorbing rim area 12.
22. The smart beverage container 10 according to any one of embodiments 17 to 21, wherein the pores of the plurality of pores have a size ranging from 30µm to 300µm.
23. The smart beverage container 10 according to any one of the preceding embodiments, wherein the thickness of the saliva absorbing rim area 12 is in the range of 1mm to 10mm.
24. The smart beverage container 10 according to any one of the preceding embodiments, wherein the height of the saliva absorbing rim area 12 is in the range of 3mm to 10mm.
25. A smart beverage container 10 according to any one of the preceding embodiments, wherein the microfluidic network 15 has a dendritic distribution, the upper end comprising a higher number of channels being connected to the saliva absorbing rim area 12 and a lower end comprising a lower number of channels being connected to the biosensor 13.
26. The smart beverage container 10 according to any one of the preceding embodiments, wherein the cross-sections of the channels are circular and/or rectangular.
27. The smart beverage container 10 according to any one of the preceding embodiments, wherein the diameters of channels being closer to the upper end are smaller than the diameters of channels being closer to the lower end.
28. The smart beverage container 10 according to any one of the preceding embodiments, wherein the diameters of the channels are equal or different.
29. The smart beverage container 10 according to any one of the preceding embodiments, wherein the diameter of each channel is in the range of 10µm to 3mm.
30. The smart beverage container 10 according to any one of the preceding embodiments, wherein the power source comprises a thermoelectric generator film 18 and/or a wireless power transfer coil 19 configured to receive power from a mobile device.
31. The smart beverage container 10 according to embodiment 30, wherein the wireless power transfer coil is configured to receive power from a mobile device through inductive coupling, capacitive coupling, magnetodynamic coupling, and/or Zenneck wave transmission.
32. The smart beverage container 10 according to embodiment 30, wherein the thermoelectric generator film 18 is configured to generate electrical power using the heat of beverage inside the container having a temperature higher than the environmental temperature outside the container.
33. The smart beverage container 10 according to any one of embodiments 30 to 32, wherein the thermoelectric generator film is based on the Seebeck effect or the Nerst effect.
34. The smart beverage container 10 according to any one of embodiments 30 to 33, wherein the thermoelectric generator film is made of a material comprising semiconductor type like bismuth telluride, lead telluride, and/or silicon germanium, inorganic clathrates, compounds of Mg and group-14 element, skutterudites, cation-substituted copper sulfide thermoelectrics, Half-Heusler alloys, silicon-germanium, sodium-cobaltate, amorphous materials, functionally graded materials, tin selenide, silicon-nanowire, and/or iron-doped with aluminum (Fe3Al) or gallium (Fe3Ga).
35. The smart beverage container 10 according to any one of embodiments 30 to 34, wherein the thickness of thermoelectric generator film 18 is in the range of 0.1mm to 2mm.
36. The smart beverage container 10 according to any one of embodiments 30 to 35, wherein the thermoelectric generator film 18 is located on the internal side of the body 11 or is located on the external side of the body 11.
37. The smart beverage container 10 according to any one of embodiments 30 to 36, wherein the body 11 comprises at least two walls 11a, 11b, wherein the biosensor 13, the communication interface 16, the microfluidic network 15, and/or the thermoelectric generator film 18 are located between two of the at least two walls 11a, 11b.
38. The smart beverage container 10 according to embodiment 37, wherein the body comprises an air gap or an insulating layer between two of the at least two walls 1 1q, 11b.
39. The smart beverage container 10 according to any one of the preceding embodiments, wherein the body 11 is made of a material comprising a biodegradable, compostable, degradable and/or recyclable material.
40. The smart beverage container 10 according to any one of the preceding embodiments, wherein the body 11 is made of a material comprising plastic (PS, PP, PET, EPS, PC), bioplastic, paper, wood, glass, ceramic, aluminum, cork and/or polycoated.
41. The smart beverage 10 container according to any one of the preceding embodiments, wherein the body 11 comprises an insulating layer at one or each of the sides of the body 11.
42. The smart beverage container 10 according to any one of the preceding embodiments, wherein the diameter of a base of the body 11 is in the range of 40mm to 90mm.
43. The smart beverage container 10 according to any one of the preceding embodiments, wherein the height of the body is in the range of 50mm to 180mm.
44. The smart beverage container 10 according to any one of the preceding embodiments, wherein the capacity of the body 11 is in the range of 200ml to 600ml.
45. The smart beverage container 10 according to any one of the preceding embodiments, further comprising a lid or a cap attached to the top edge of the body 11.
46. The smart beverage container 10 according to any one of the preceding embodiments, is one of a cup, a bottle, or a thermos.
47. A computer-implemented method 100 for visualizing a saliva analysis result, comprising
   receiving 110, via a user interface, a selection of one or more test protocols selected by the user from a plurality of test protocols;
   enabling 120 the communication with a communication interface of a smart beverage container according to any one of embodiments 1 to 46;
   receiving 130, from the communication interface, results of a saliva analysis processed by a sensor of the beverage container; and
   visualizing 140 the results on a display device.
48. A method for using a smart beverage container for analyzing saliva, comprising providing a smart beverage container according to any one of the preceding embodiments 1 to 13;
   pouring hot beverage into the container;
   removing a protective sealing film from a saliva absorbing rim area;
   drinking a sip of the beverage;
   bringing a mobile device in contact with a communication interface coupled to the body of the container; and
   reading the result directly from a display device attached to the body of the container and/or
   and reading result from a display of a mobile device.
49. A method of manufacturing a smart beverage container, comprising
   providing a body 11 comprising a top edge with a saliva absorbing rim area 12 configured to collect saliva;
   coupling a biosensor 13 to the body 11 configured to analyze saliva;
   coupling a microfluidic network 15 between the saliva absorbing rim area 12 and the biosensor 13 configured to direct the collected saliva to the biosensor;
   coupling a power source 18, 19 to the body 11 configured to provide electrical power, and
   coupling a communication interface 16 to the body 11.

## Claims

1. A smart beverage container (10) for analyzing saliva, comprising
a body (11) comprising a top edge with a saliva absorbing rim area (12) configured to collect saliva of a user;
a biosensor (13) coupled to the body (11) and configured to receive power from a power source (18, 19); and
a microfluidic network (15) between the saliva absorbing rim area and the biosensor (13), wherein the microfluidic network (15) comprises a plurality of channels configured to direct the collected saliva to the biosensor (13);
wherein the biosensor (13) is configured to analyze saliva and to provide the result of the analysis to a communication interface (16).

2. The smart beverage container (10) according to claim 1, wherein the communication interface (16) comprises a near-field communication chip.

3. The smart beverage container (10) according to claim 1 or 2, further comprising a display device (17) located on an external side of the body (11) and configured to visualize the result of the analysis.

4. The smart beverage container (10) according to claim 3, wherein the display device (17) comprises an electronic paper display.

5. The smart beverage container (10) according to any one of the preceding claims, wherein the biosensor (13) is configured to detect one or more biomarkers, viruses, and/or bacteria.

6. The smart beverage container (10) according to any one of the preceding claims, wherein the biosensor (13) is one of an amperometric biosensor, an organic electrochemical transistor (OECT), or an all-polymer micrometer-scale transistor biosensor.

7. The smart beverage container (10) according to any one of the preceding claims, wherein the one or more biomarkers comprise endocrine biomarkers like cortisol, testosterone, and/or insulin, immunologic biomarkers like IgA, IgM, and/or IgG, inflammatory biomarkers like cytokines, proteins like enzymes or antibodies, infectious or pathogen RNA, metabolites like vitamins, and/or cancer biomarkers.

8. The smart beverage container (10) according to any one of the preceding claims, wherein the saliva absorbing rim area (12) comprises a plurality of pores microfluidically coupled to an upper end of the channels of the microfluidic network.

9. A smart beverage container (10) according to any one of the preceding claims, wherein the microfluidic network (15) has a dendritic distribution, the upper end comprising a higher number of channels being connected to the saliva absorbing rim area (12) and a lower end comprising a lower number of channels being connected to the biosensor (13).

10. The smart beverage container (10) according to any one of the preceding claims, wherein the power source comprises a thermoelectric generator film (18) and/or a wireless power transfer coil (19) configured to receive power from a mobile device (20).

11. The smart beverage container (10) according to claim 10, wherein the thermoelectric generator film (18) is configured to generate electrical power using the heat of beverage inside the container having a temperature higher than the environmental temperature outside the container.

12. The smart beverage container (10) according to claims 10 or 11, wherein the thermoelectric generator film (18) is located on the internal side of the body (11) or is located on the external side of the body (11).

13. The smart beverage container (10) according to any one of claims 10 to 12, wherein the body (11) comprises at least two walls 11a, 11b, wherein the biosensor (13), the communication interface 16), the microfluidic network 15, and/or the thermoelectric generator film (18) are located between two of the at least two walls.

14. A computer-implemented method (100) for visualizing a saliva analysis result, comprising
receiving (110), via a user interface, a selection of one or more test protocols selected by the user from a plurality of test protocols;
enabling (120) the communication with a communication interface of a smart beverage container according to any one of claims 1 to 13;
receiving (130), from the communication interface, results of a saliva analysis processed by a sensor of the beverage container; and
visualizing (140) the results on a display device.

15. A method of manufacturing a smart beverage container (10), comprising
providing a body (11) comprising a top edge with a saliva absorbing rim area (12) configured to collect saliva;
coupling a biosensor (13) to the body (11) configured to analyze saliva;
coupling a microfluidic network (15) between the saliva absorbing rim area (12) and the biosensor (13) configured to direct the collected saliva to the biosensor;
coupling a power source (18, 19) to the body (11) configured to provide electrical power, and
coupling a communication interface (16) to the body (11).
